# FASCICULE DE BREVET EUROPEEN

(11) **EP 2 716 262 B1**
(45) Date de publication et mention de la délivrance du brevet: **13.05.2015**
(21) Numéro de dépôt: 13187656.7
(22) Date de dépôt: 08.10.2013
(51) Int. Cl.: A61F 2/44

(54) **Prothese de disque intervertebral**
Bandscheibenprothese
Intervertebral disc prosthesis

(30) Priorité: 08.10.2012 FR 1259556
(43) Date de publication de la demande: 09.04.2014
(73) Titulaire: Medicrea International, 01700 Neyron (FR)
(72) Inventeur: Mosnier, Thomas, 38280 ANTHON (FR); Sournac, Denys, 01600 REYRIEUX (FR)
(74) Mandataire: Jeannet, Olivier

(56) Documents cités:
- EP-A2- 0 950 389
- US-A1- 2007 191 958
- US-A1- 2010 016 970
- US-A1- 2012 116 516

## Description

La présente invention concerne une prothèse de disque intervertébral, notamment lombaire.

Une prothèse connue de disque intervertébral comprend deux plateaux destinés à être fixés aux vertèbres à traiter et un élément souple interposé entre ces plateaux, pour permettre le positionnement de ces plateaux l'un par rapport à l'autre et l'amortissement des mouvements de ces plateaux, exercés par les vertèbres. Cet élément souple peut faire partie d'un noyau incluant deux coques assemblées, l'élément souple étant contenu dans un espace délimité par ces coques ; chacune de ces dernières forme une face articulaire coopérant avec une face articulaire conjuguée que comprend le plateau correspondant.

Les mouvements exercés par les vertèbres sont complexes (rapprochement/éloignement mutuel, pivotement, rotation, translation latérale et/ou antéropostérieure, ces différents mouvements étant le plus fréquemment combinés), et sont répétés de nombreuses fois. Il s'avère que les prothèses existantes présentent toutes un problème de résistance dans le temps de leurs éléments souples, les mouvements complexes et répétés générant de l'usure et des cisaillements aux éléments souples.

La présente invention a pour objectif principal de fournir une prothèse remédiant à ce problème essentiel.

Un autre objectif de l'invention est d'atteindre ce but au moyen d'une prothèse reproduisant fidèlement les mouvements que permettent les disques vertébraux naturels.

Un autre objectif encore de l'invention est d'atteindre ces buts au moyen d'une prothèse restant de conception relativement simple.

Le document US 2012/116516 décrit en outre une prothèse comprenant deux pièces s'étendant principalement dans un plan, et un élément souple interposé entre ces deux pièces ; cet élément souple comprend, sur deux côtés opposés, deux zones de contact avec ces pièces et permet, par sa déformation élastique, des mouvements de rapprochement/éloignement desdites pièces selon une direction perpendiculaire audit plan ; chaque pièce présente une face de forme arrondie destinée à venir en contact avec la zone de contact correspondante de l'élément souple ; les pièces présentent des moyens de guidage de leurs mouvements de rapprochement/éloignement l'une par rapport à l'autre, ces moyens de guidage permettant uniquement ces mouvements de rapprochement/éloignement des pièces.

Le préambule de la revendication 1 expose les caractéristiques de la prothèse selon l'invention qui sont connues par ce document antérieur.

Selon l'invention,
- l'élément souple est une bille sphérique ;
- lesdites faces de forme arrondie sont formées par des cuvettes en calottes de sphères creuses aménagées sensiblement au centre desdites pièces, recevant cet élément souple ; et
- lesdites pièces forment un espace libre entourant l'ensemble de la périphérie de l'élément souple, en dehors desdites zones de contact.

Ainsi, conformément à l'invention, l'élément souple est en contact avec lesdites pièces uniquement par lesdites zones de contact et, en dehors desdites zones de contact, est entièrement entouré par ledit espace libre, sur l'ensemble de sa périphérie. Cet élément souple est ainsi apte à se déformer lorsque les pièces sont rapprochées l'une de l'autre, en étant guidé par lesdites cuvettes en calottes de sphères creuses, et vient fluer élastiquement dans ledit espace libre. Les moyens de guidage que forment les pièces limitent le mouvement de ces pièces à un pur mouvement de rapprochement/éloignement des pièces, et préservent ainsi l'élément souple de tout effort de cisaillement.

Cette combinaison de moyens permet que, dans la prothèse selon l'invention, l'élément souple travaille uniquement en compression, sans rencontrer aucune paroi ni aucune arête vive lors de sa déformation et sans aucun effort de cisaillement, ce qui élimine toute risque d'autre déformation susceptible de le détériorer.

Il sera compris que, par "deux pièces s'étendant principalement dans un plan", on entend des pièces ayant des formes plus ou moins aplaties, avec une longueur et une largeur s'étendant dans ledit plan, nettement supérieures à l'épaisseur de ces pièces, comme peuvent l'être les plateaux d'une prothèse de disque vertébral ou les coques d'un noyau d'une telle prothèse.

Avantageusement, le bord délimitant la périphérie de chaque cuvette est arrondi de telle sorte qu'il existe une transition douce entre la face délimitant la cuvette et la face environnant cette cuvette.

Ce bord n'est ainsi pas marqué et n'est donc pas agressif pour l'élément souple.

De préférence, la profondeur de chaque cuvette est de l'ordre de 15 % du diamètre de la bille.

De préférence, la périphérie dudit espace libre est délimitée par face courbe, de courbure constante sur l'ensemble de cette périphérie.

Lesdites pièces pourraient être les plateaux de la prothèse eux-mêmes. De préférence, ces pièces sont les deux coques d'un noyau que comprend la prothèse, destiné à être placé entre ces plateaux, ces coques comprenant des moyens d'assemblage de l'une à l'autre permettant, dans une position d'éloignement relatif de ces coques, une légère mise en compression de l'élément souple afin de maintenir ce dernier en position entre lesdites faces de forme arrondie.

Par "légère mise en compression de l'élément souple ", il faut entendre une mise en compression inférieure à 15 % de la capacité totale de compression de cet élément souple.

Selon une forme de réalisation préférée de l'invention dans ce cas, lesdits moyens d'assemblage sont constitués par deux parois périphériques dont l'une est solidaire d'une coque et l'autre est solidaire de l'autre coque, ces parois périphériques comprenant des dents ou rebords d'encliquetage d'une paroi avec l'autre.

De préférence, dans ce cas, lesdits moyens de guidage sont formés par une face périphérique extérieure de l'une desdites parois périphériques, venant, lorsque les coques sont assemblées, à proximité immédiate d'une face périphérique intérieure de l'autre paroi périphérique.

L'invention sera bien comprise, et d'autres caractéristiques et avantages de celle-ci apparaîtront, en référence au dessin schématique annexé, représentant, à titre d'exemple non limitatif, une forme de réalisation préférée de la prothèse concernée.
La figure 1 en est une vue en perspective ;
la figure 2 en est une vue similaire à la figure 1, à échelle agrandie et avec coupe antéro-postérieure médiane ;
la figure 3 est une vue à échelle encore agrandie, en coupe médiane, d'un noyau que comprend la prothèse, avant assemblage ;
la figure 4 en est une vue similaire à la figure 3, après assemblage, dans un état d'éloignement relatif de deux coques formant ce noyau, et
la figure 5 en est une vue similaire à la figure 4, dans un état de rapprochement relatif de ces deux coques.

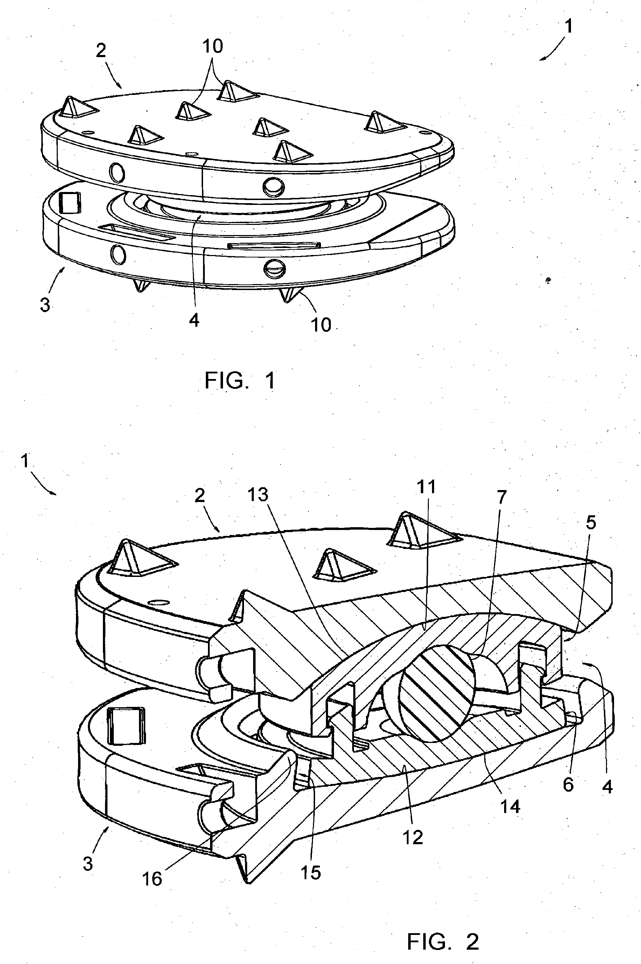

La figure 1 représente une prothèse 1 de disque intervertébral, notamment lombaire, qui comprend deux plateaux 2, 3 destinés à être fixés aux vertèbres à traiter et un noyau 4 interposé entre ces plateaux 2, 3. En référence aux figures 2 à 5, il apparaît que ce noyau 4 est formé par deux coques 5, 6 assemblées, contenant une bille 7 en un matériau souple.

Les plateaux 2, 3, de même que les coques 5, 6, ont des formes plus ou moins aplaties, c'est-à-dire qu'ils s'étendent principalement dans un plan, avec une longueur et une largeur nettement supérieures à l'épaisseur de ces pièces.

Les plateaux 2, 3 comprennent des plots 10 faisant saillie de leurs faces destinés à venir contre les plateaux des vertèbres à traiter, qui permettent d'assurer l'immobilisation de ces plateaux à ces vertèbres. Ils comprennent également des faces articulaires courbes 11, 12, en portions de sphères, destinées à coopérer avec des faces articulaires conjuguées 13, 14 que forment les coques 5, 6. Il apparaît sur la figure 2 que la face articulaire 11 du plateau supérieur 2 a une courbure plus prononcée que la face articulaire 12 du plateau inférieur 3. Cette dernière est aménagée dans le fond d'une cavité circulaire 15 creusée dans l'épaisseur du plateau 3, délimitée par une paroi périphérique 16.

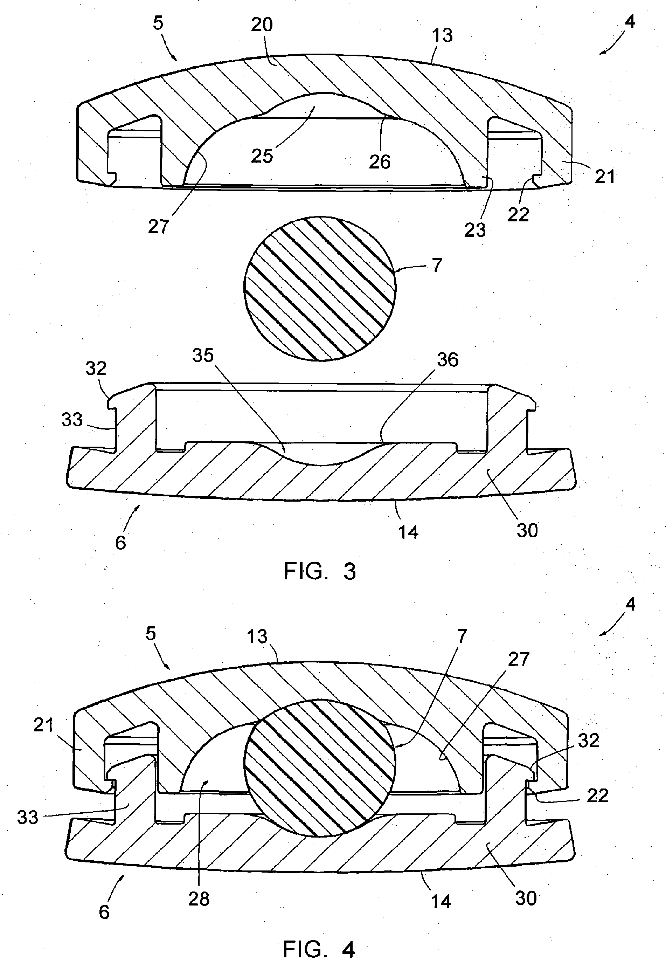

En référence à la figure 3, il apparaît que la coque supérieure 5 comprend une paroi principale 20 formant extérieurement la face articulaire 13, une paroi périphérique externe 21 comprenant un rebord interne d'encliquetage 22 et une paroi périphérique interne 23 formant une face périphérique interne courbe 27, de courbure constante sur l'ensemble de sa périphérie, et une face périphérique externe droite. Sensiblement en son centre, la coque 5 comprend une cuvette interne 25 en calotte de sphère creuse, aménagée dans la paroi 20, de rayon de courbure correspondant sensiblement à celui de la bille 7 et de profondeur de l'ordre de 15% du diamètre de celui de cette bille. Le bord 26 délimitant la périphérie de cette cuvette 25 est arrondi de telle sorte qu'il existe une transition douce entre la face délimitant la cuvette 25 et la base de ladite face périphérique interne courbe, environnant cette cuvette.

Comme le montre la figure 4, la face délimitée par la cuvette 25 est destinée à venir en contact avec la calotte correspondante de la bille 7. La face périphérique interne courbe délimitée paroi périphérique interne 23 s'étend à distance de la bille 7 et délimite, à l'état d'assemblage, un espace libre 28 entourant la totalité de la périphérie de la bille 7.

La coque inférieure 6 comprend une paroi principale 30, une cuvette 35 et un bord 36 homologues respectivement à la paroi principale 20, à la cuvette 25 et au bord 26. Elle comprend une paroi périphérique interne 33 formant une face périphérique interne droite et comprenant un rebord externe d'encliquetage 32.

En référence à la figure 4, il apparaît qu'à l'état d'assemblage du noyau 4, la bille 7 prend place dans les cuvettes 25, 35 ; ladite face périphérique externe droite vient à proximité immédiate de ladite face périphérique interne droite, de sorte que les parois 23, 33 réalisent un guidage du mouvement de rapprochement / éloignement des coques 5, 6 ; les rebords d'encliquetage 22, 32 viennent en coopération l'un avec l'autre pour réaliser l'assemblage des coques 5, 6. Comme le montre cette figure 4, la bille 7 est légèrement comprimée entre les coques 5, 6 lorsque ces rebords 22, 32 sont au contact l'un de l'autre, de sorte que cette bille 7 est fermement maintenue en position entre les coques 5, 6, dans les cuvettes 25, 35.

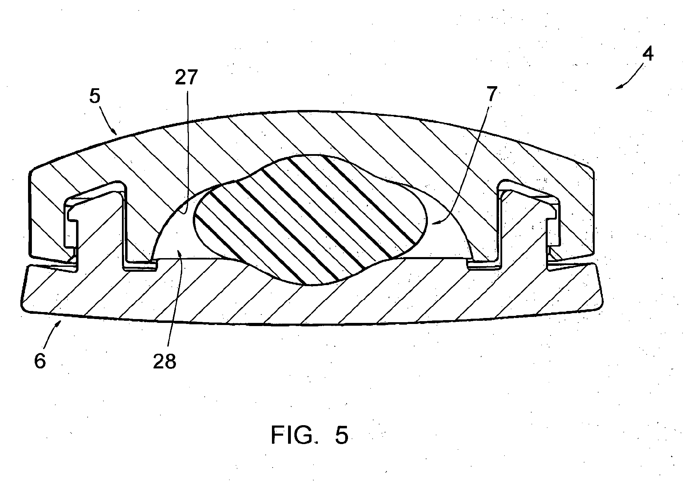

Dans cet état d'assemblage du noyau 4, l'espace libre 28 entoure l'ensemble de la périphérie de la bille 7 en dehors des zones de contact de cette bille 7 avec les faces arrondies délimitant les cuvettes 25, 35, de sorte que, ainsi que le montre la figure 5, la bille 7 est apte à se déformer dans cet espace 28 lorsque les coques sont rapprochées l'une de l'autre.

La prothèse 1 a ainsi, par rapport aux prothèses homologues existantes, les avantages déterminants :
- d'avoir un élément souple résistant dans le temps nonobstant les mouvements complexes et répétés exercés par les vertèbres ;
- d'atteindre ce but au moyen d'une prothèse reproduisant fidèlement les mouvements que permettent les disques vertébraux naturels ;
- d'atteindre ce but au moyen d'une prothèse restant de conception relativement simple.

L'invention a été décrite ci-dessus en référence à une forme de réalisation donnée à titre d'exemple. Il va de soi qu'elle n'est pas limitée à cette forme de réalisation mais qu'elle s'étend à toutes les autres formes de réalisation couvertes par les revendications annexées.

## Revendications

1. Prothèse (1) de disque intervertébral, comprenant deux pièces (5, 6) s'étendant principalement dans un plan et un élément souple (7) interposé entre ces deux pièces (5, 6) ; cet élément souple (7) est une bille sphérique et comprend, sur deux côtés opposés, deux zones de contact avec ces pièces (5, 6) et permet, par sa déformation élastique, des mouvements de rapprochement/éloignement desdites pièces (5, 6) selon une direction perpendiculaire audit plan ; chaque pièce (5, 6) présente une face de forme arrondie destinée à venir en contact avec la zone de contact correspondante de l'élément souple (7) ; les pièces (5, 6) présentent des moyens (23, 33) de guidage de leurs mouvements de rapprochement/éloignement l'une par rapport à l'autre, ces moyens de guidage (23, 33) permettant uniquement ces mouvements de rapprochement/éloignement des pièces (5, 6) ;
**caractérisée en ce que** :
- lesdites faces de forme arrondie sont formées par des cuvettes (25, 35) en calottes de sphères creuses aménagées sensiblement au centre desdites pièces (5, 6), recevant cet élément souple (7) ; et
- lesdites pièces (5, 6) forment un espace libre (28) entourant l'ensemble de la périphérie de l'élément souple (7), en dehors desdites zones de contact.

2. Prothèse (1) selon la revendication 1, **caractérisée en ce que** le bord (26, 36) délimitant la périphérie de chaque cuvette (25, 35) est arrondi de telle sorte qu'il existe une transition douce entre la face délimitant la cuvette (25, 35) et la face environnant cette cuvette.

3. Prothèse (1) selon la revendication 1 ou la revendication 2, **caractérisée en ce que** la profondeur de chaque cuvette (25, 35) est de l'ordre de 15 % du diamètre de la bille (7).

4. Prothèse (1) selon l'une des revendications 1 à 3, **caractérisée en ce que** la périphérie dudit espace libre est délimitée par face courbe, de courbure constante sur l'ensemble de cette périphérie.

5. Prothèse (1) selon l'une des revendications 1 à 4, **caractérisée en ce que** lesdites pièces sont les plateaux de la prothèse destinés à être fixés aux plateaux des vertèbres à traiter.

6. Prothèse (1) selon l'une des revendications 1 à 4, **caractérisée en ce que** lesdites pièces sont les deux coques (5, 6) d'un noyau (4) que comprend la prothèse, placé entre les plateaux (2, 3) de cette prothèse destinés à être fixés aux plateaux des vertèbres à traiter, ces coques (5, 6) comprenant des moyens d'assemblage (21, 22, 32, 33) de l'une à l'autre permettant, dans une position d'éloignement relatif de ces coques, une légère mise en compression de l'élément souple (7) afin de maintenir ce dernier en position entre lesdites faces de forme arrondie.

7. Prothèse (1) selon la revendication 6, **caractérisée en ce que** lesdits moyens d'assemblage sont constitués par deux parois périphériques (21, 33) dont l'une est solidaire d'une coque (5) et l'autre est solidaire de l'autre coque (6), ces parois périphériques (21, 33) comprenant des dents ou rebords d'encliquetage (22, 32) d'une paroi avec l'autre.

8. Prothèse (1) selon la revendication 7, **caractérisée en ce que** lesdits moyens de guidage sont formés par une face périphérique extérieure de l'une desdites parois périphériques (23), venant, lorsque les coques (5, 6) sont assemblées, à proximité immédiate d'une face périphérique intérieure de l'autre paroi périphérique (33).

## Patentansprüche

1. Bandscheibenprothese (1), die zwei Teile (5, 6), die sich hauptsächlich in einer Ebene erstrecken, und ein nachgiebiges Element (7) umfasst, das zwischen diesen zwei Teilen (5, 6) angeordnet ist; wobei dieses nachgiebige Element (7) eine sphärische Kugel ist und auf zwei entgegengesetzten Seiten zwei Berührungsbereiche mit diesen Teilen (5, 6) aufweist und durch seine elastische Verformung Bewegungen zur Annäherung/Entfernung der Teile (5, 6) in einer Richtung senkrecht zu der Ebene ermöglicht; wobei jedes Teil (5, 6) eine Seite mit abgerundeter Form aufweist, die dazu bestimmt ist, mit dem entsprechenden Berührungsbereich des nachgiebigen Elements (7) in Berührung zu gelangen; wobei die Teile (5, 6) Mittel (23, 33) zur Führung ihrer Bewegungen zur Annäherung/Entfernung in Bezug zueinander aufweisen, wobei diese Führungsmittel (23, 33) einzig diese Bewegungen zur Annäherung/Entfernung der Teile (5, 6) zulassen;
**dadurch gekennzeichnet, dass**:
- die Seiten mit abgerundeter Form durch Schalen (25, 35) in der Form von hohlen Kugelkappen gebildet sind, die im Wesentlichen in der Mitte der Teile (5, 6) eingerichtet sind, die dieses nachgiebige Element (7) aufnehmen; und
- die Teile (5, 6) einen freien Raum (28) bilden, der die Gesamtheit des Umfangs des nachgiebigen Elements (7) außerhalb der Berührungsbereiche umgibt.

2. Prothese (1) nach Anspruch 1, **dadurch gekennzeichnet, dass** der Rand (26, 36), der den Umfang von jeder Schale (25, 35) abgrenzt, derart abgerundet ist, dass ein sanfter Übergang zwischen der Seite, die die Schale (25, 35) abgrenzt, und der Seite, die diese Schale umgibt, besteht.

3. Prothese (1) nach Anspruch 1 oder Anspruch 2, **dadurch gekennzeichnet, dass** die Tiefe jeder Schale (25, 35) in der Größenordnung von 15% des Durchmessers der Kugel (7) ist.

4. Prothese (1) nach einem der Ansprüche 1 bis 3, **dadurch gekennzeichnet, dass** der Umfang des freien Raumes durch eine gekrümmte Seite mit über die Gesamtheit dieses Umfangs konstanter Krümmung abgegrenzt ist.

5. Prothese (1) nach einem der Ansprüche 1 bis 4, **dadurch gekennzeichnet, dass** die Teile die Platten der Prothese sind, die dazu bestimmt sind, an den Platten der zu behandelnden Wirbel befestigt zu werden.

6. Prothese (1) nach einem der Ansprüche 1 bis 4, **dadurch gekennzeichnet, dass** die Teile die zwei Schalen (5, 6) eines Kerns (4) sind, den die Prothese umfasst, der zwischen den Platten (2, 3) dieser Prothese untergebracht ist, die dazu bestimmt sind, an den Platten der zu behandelnden Wirbel befestigt zu werden, wobei diese Schalen (5, 6) Mittel zum Zusammenbau (21, 22, 32, 33) aneinander umfassen, die in einer entfernten Stellung in Bezug zu diesen Schalen ein leichtes Zusammendrücken des nachgiebigen Elements (7) ermöglichen, um dieses letztere zwischen den Seiten der abgerundeten Form an Ort und Stelle zu halten.

7. Prothese (1) nach Anspruch 6, **dadurch gekennzeichnet, dass** die Mittel zum Zusammenbau aus zwei Umfangswänden (21, 33) gebildet sind, von denen die eine fest mit einer Schale (5) verbunden ist und die andere fest mit der anderen Schale (6) verbunden ist, wobei diese Umfangswände (21, 33) Zähne oder Ränder (22, 32) zum Einrasten einer Wand mit der anderen umfassen.

8. Prothese (1) nach Anspruch 7, **dadurch gekennzeichnet, dass** die Führungsmittel durch eine äußere Umfangsseite von einer der Umfangswände (23) gebildet sind, die, wenn die Schalen (5, 6) zusammengebaut werden, in unmittelbare Nähe einer inneren Umfangsseite der anderen Umfangswand (33) gelangen.

## Claims

1. Intervertebral disc prosthesis (1), comprising two pieces (5, 6) extending mainly in a plane and a flexible element (7) interposed between these two pieces (5, 6); this flexible element (7) is a spherical ball and has, on two opposite sides, two contact zones in contact with these pieces (5, 6) and allows, through its elastic deformation, movements by which said pieces (5, 6) come closer or come apart from one another in a direction perpendicular to said plane; each part (5, 6) has a rounded surface adapted to come into contact with the corresponding contact zone of the flexible element (7); the pieces (5, 6) have guide means (23, 33) for guiding their movements claser/apart from one another, said guide means (23, 33) only allowing these movements claser/apart of the pieces (5, 6);
**characterized in that**:
- said rounded surfaces are formed by cups (25, 35) in the form of hollow spherical caps arranged substantially at the center of said pieces (5, 6), receiving the flexible element (7); and
- said pieces (5, 6) form a free space (28) surrounding the entire periphery of the flexible element (7) outside of said contact zones.

2. Prosthesis (1) according to claim 1, **characterized in that** the edge (26, 36) defining the periphery of each cup (25, 35) is rounded so that there is a smooth transition between the face delimiting the cup (25, 35) and the surface surrounding this cup.

3. Prosthesis (1) according to claim 1 or claim 2, **characterized in that** the depth of each cup (25, 35) is of the order of 15% of the diameter of the ball (7).

4. Prosthesis (1) according to one of claims 1 to 3, **characterized in that** the periphery of said free space is delimited by a curved surface of constant curvature over the whole periphery.

5. Prosthesis (1) according to one of claims 1 to 4, **characterized in that** said pieces are the plates of the prosthesis intended to be fixed to the plates of the vertebrae.

6. Prosthesis (1) according to one of claims 1 to 4, **characterized in that** said pieces are the two shells (5, 6) of a core (4) that the prosthesis comprises, placed between the plates (2, 3) of the prosthesis intended to be fixed to the plates of the vertebrae to be treated, these shells (5, 6) comprising joining means (21, 22, 32, 33) for joining one to the other allowing, in a relative apart position of these shells, a slight compression of the flexible element (7) so as to keep the latter in position between said rounded surfaces.

7. Prosthesis (1) according to claim 6, **characterized in that** said joining means are constituted by two peripheral walls (21, 33), of which one is integral with a shell (5) and the other is integral with the other shell (6), said peripheral walls (21, 33) comprising snap teeth or flanges (22, 32) for snapping engagement of one wall with the other.

8. Prosthesis (1) according to claim 7, **characterized in that** said guide means are formed by an outer peripheral surface of one of said peripheral walls (23), coming, when the shells (5, 6) are assembled, in the immediate vicinity of an inner peripheral surface of the other peripheral wall (33).
